# FASCICULE DE BREVET EUROPEEN

(11) **EP 0 876 434 B1**
(45) Date de publication et mention de la délivrance du brevet: **26.01.2000**
(21) Numéro de dépôt: 96939968.2
(22) Date de dépôt: 22.11.1996
(51) Int. Cl.: C09C 3/06, C09C 1/00, C09C 1/12, C09C 1/38, C09C 1/32, C09D 7/12, C04B 33/14, C08K 9/02, D21H 19/38, D21H 17/67, C09D 11/00

(54) **COMPOSES SOUFRES REVETUS D'UN COMPOSE DE ZINC UTILISABLES COMME PIGMENTS**
MIT EINER ZINKVERBINDUNG BESCHICHTETE SCHWEFELVERBINDUNGEN, VERWENDBAR ALS PIGMENTE
SULPHUR COMPOUNDS COATED WITH A ZINC COMPOUND FOR USE AS PIGMENTS

(30) Priorité: 24.11.1995 FR 9513973
(43) Date de publication de la demande: 11.11.1998
(73) Titulaire: RHODIA CHIMIE, 92408 Courbevoie Cédex (FR)
(72) Inventeur: BUSNOT, Sylvain, F-60660 Maysel (FR); MACAUDIERE, Pierre, F-92600 Asnières-sur-Seine (FR)
(74) Mandataire: Dubruc, Philippe
(86) Numéro de dépôt international: FR9601853
(87) Numéro de publication internationale: WO9720002

(56) Documents cités:
- EP-A- 0 203 838
- EP-A- 0 545 746
- EP-A- 0 620 254
- EP-A- 0 628 608
- EP-A- 0 680 930
- DE-A- 4 404 953
- US-A- 4 331 706

## Description

La présente invention concerne une composition à base d'un composé soufré, de zinc et éventuellement d'un oxyde transparent, son procédé de préparation et son utilisation comme pigment colorant.

Les pigments minéraux de coloration sont déjà largement utilisés dans de nombreuses industries notamment dans celles des peintures, des matières plastiques et des céramiques. Parmi ces pigments, on trouve un certain nombre de compositions contenant du soufre. Notamment, des produits à base de sulfures de terres rares ont déjà été proposés par la Demanderesse comme substituts à des pigments comprenant des métaux à toxicité réputée très élevée comme, notamment, le cadmium, le plomb, le chrome et le cobalt dont l'emploi devient de plus en plus sévèrement réglementé. Des compositions à base de sesquisulfures de terre rare et d'éléments alcalins ont ainsi été décrits dans EP-A-545746.

Outre le problème de la toxicité dans certains cas, les pigments à base de soufre présentent d'une manière générale l'inconvénient de dégager de l'H₂S dans certaines applications, par exemple lors de leur incorporation dans des milieux contenant des traces d'eau, comme les polymères, et tout particulièrement lorsque cette incorporation se fait à une température relativement élevée, par exemple d'au moins 200°C.

Il existe donc un besoin en pigments à base de soufre dont le dégagement d'H₂S soit minimisé.

Le revêtement d'un support à base d'au moins un composé soufré par un composé du zinc est divulgué dans DE-A-4 404 953.

L'objet de l'invention est de procurer de tels pigments améliorés.

Dans ce but, la composition selon l'invention est caractérisée en ce qu'elle comprend :
- un support à base d'au moins un composé soufré;
- un composé du zinc, déposé sur le support et obtenu par réaction d'un précurseur du zinc avec de l'ammoniaque et/ou un sel d'ammonium.

Selon un mode de réalisation particulier de l'invention, la composition de l'invention est caractérisée en ce qu'elle comprend en outre une couche à base d'au moins un oxyde transparent déposée à la surface du support.

Selon un autre mode de réalisation de l'invention, la composition est caractérisée en ce qu'elle comprend en outre des atomes de fluor.

L'invention concerne aussi un premier procédé de préparation d'une composition du type précédent, qui est caractérisé en ce qu'on met en contact le support, un précurseur du zinc, de l'ammoniaque et/ou un sel d'ammonium et, le cas échéant, un précurseur de l'oxyde transparent et un agent fluorant, et on dépose le composé de zinc sur le support et, le cas échéant, on précipite l'oxyde transparent sur ledit support.

L'invention concerne en outre un deuxième procédé de préparation d'une composition du type précédent qui est caractérisé en ce qu'on soumet le support à un traitement de fluoration dans un premier temps, puis, dans un deuxième temps, on met en contact ledit support ainsi traité, un précurseur du zinc, de l'ammoniaque et/ou un sel d'ammonium et, le cas échéant un précurseur de l'oxyde transparent, et on dépose le composé de zinc sur le support et, le cas échéant, on précipite l'oxyde transparent sur ledit support.

L'invention concerne encore un troisième procédé de préparation d'une composition du type précédent qui est caractérisé en ce que, dans une première étape, on met en contact le support, un précurseur du zinc, de l'ammoniaque et/ou un sel d'ammonium et, le cas échéant un précurseur de l'oxyde transparent, et on dépose le composé de zinc sur le support et, le cas échéant, on précipite l'oxyde transparent sur ledit support puis, dans une deuxième étape, on effectue le traitement de fluoration.

L'invention concerne par ailleurs un quatrième procédé de préparation d'une composition du type précédent qui est caractérisé en ce que, dans une première étape, on met en contact le support et un précurseur de l'oxyde transparent et on précipite l'oxyde transparent sur ledit support puis, dans une deuxième étape, on met en contact le support ainsi obtenu avec un précurseur du zinc, de l'ammoniaque et/ou un sel d'ammonium et on dépose le composé de zinc sur le support.

Enfin, l'invention concerne l'utilisation comme pigment colorant d'une composition du type précédent ou telle qu'obtenue par un des procédés du type décrit ci-dessus.

D'autres caractéristiques, détails et avantages de l'invention apparaîtront encore plus complètement à la lecture de la description qui va suivre, ainsi que des divers exemples concrets mais non limitatifs destinés à l'illustrer.

Pour la suite de la description, on entend par terre rare les éléments du groupe constitué par l'yttrium et les éléments de la classification périodique de numéro atomique compris inclusivement entre 57 et 71.

La composition selon l'invention comporte tout d'abord un support, formant noyau, à base d'au moins un composé soufré. Ce composé pourra être notamment tout composé utilisable pour ses qualités pigmentaires. Ce composé soufré peut être en particulier un sulfate ou un sulfure. On peut citer ici, comme exemple de sulfure, le sulfure ou le sulfoséléniure de cadmium, le sulfure de mercure. On peut aussi mentionner les pigments du type "ultramarine" violet 15 CI 77007 et bleu 29 CI 77007 selon la classification du Colour Index.

Selon une variante préférée, lorsqu'on cherche des compositions à base d'éléments non toxiques, on choisit le support parmi les produits déjà décrits par la Demanderesse. Ainsi, le support peut être à base d'un sulfure de terre rare du type Ln₂S₃, Ln étant la terre rare, comme décrit dans EP-A-203838.

Ce support peut être aussi un sulfure de terre rare et d'alcalin. Il peut s'agir plus précisément d'un sulfure de formule ALnS₂ dans laquelle A représente au moins un alcalin et Ln au moins une terre rare. On peut citer plus particulièrement ceux de formules suivantes : KLaS₂, NaCeS₂.

Selon une autre variante préférée, le sulfure est un sesquisulfure de terre rare qui contient au moins un élément alcalin et/ou alcalino-terreux dont une partie au moins est incluse dans le réseau cristallin dudit sesquisulfure. On pourra se référer à la demande de brevet européen EP-A-545746 dont l'enseignement est incorporé ici. On peut rappeler pour cette variante que l'élément alcalin peut être choisi notamment parmi le lithium, le sodium ou le potassium. Bien entendu, le sulfure ou sesquisulfure peut comprendre plusieurs éléments alcalins.

L'élément alcalin ou alcalino-terreux est inclus au moins en partie dans le réseau cristallin du sulfure ou sesquisulfure. Selon une autre variante, l'élément alcalin ou alcalino-terreux est inclus essentiellement ou totalement dans le réseau cristallin.

Le sesquisulfure peut posséder notamment une structure cristallographique cubique de type Th₃P₄, qui présente des lacunes au niveau du réseau des cations; cette structure lacunaire peut être symbolisée en donnant aux sesquisulfures la formule M_{10,66} [ ]_{1,33} S₁₆ (voir notamment à ce sujet, W.H. ZACHARIASEN, "Crystal Chemical Studies of the 5f-Series of Elements. The Ce₂S₃-Ce₃S₄ Type of Structure", Acta Cryst., (1949), 2, 57).

Les éléments alcalins ou alcalino-terreux peuvent être introduits dans ces lacunes cationiques, jusqu'à saturation ou non de ces dernières. La présence de cet élément au sein du sulfure ou sesquisulfure peut être mise en évidence par simple analyse chimique. Par ailleurs, les analyses en diffraction X montrent qu'il y a conservation de la phase cristalline en Th₃P₄ du sesquisulfure, avec dans certains cas, une modification plus ou moins importante des paramètres de maille, fonction à la fois de la nature et de la quantité de l'élément alcalin ou alcalino-terreux introduit.

Généralement, la quantité d'élément alcalin ou alcalino-terreux est d'au plus 50% de la quantité molaire en terre rare du sulfure ou du sesquisulfure .

Selon une autre caractéristique préférée, la quantité molaire en alcalin ou alcalino-terreux est au moins égale à 0,1%, et avantageusement comprise entre 5% et 50% et plus particulièrement 5 et 20%, de la quantité molaire en terre rare.

Dans cette variante comprenant un sesquisulfure, la terre rare peut être plus particulièrement le cérium ou le lanthane. Encore plus particulièrement, le sesquisulfure de terre rare est un sesquisulfure Ce₂S₃ γ cubique.

On pourra encore citer comme sulfures de terre rare utilisables comme support dans le cadre de la présente invention, ceux décrits dans la demande de brevet européen EP-A-680930 dont l'enseignement est incorporé ici. Ces sulfures de terre rare comprennent au moins un élément alcalin et ils sont constitués de grains monocristallins entiers de taille moyenne d'au plus 1,5µm. Ils sont obtenu par un procédé dans lequel on met en présence au moins un carbonate ou un hydroxycarbonate de terre rare avec au moins un composé d'un élément alcalin et on les chauffe en présence d'au moins un gaz choisi parmi le sulfure d'hydrogène ou le sulfure de carbone. Ces produits présentent par ailleurs une taille moyenne (granulométrie CILAS) généralement inférieure à 2µm, plus particulièrement comprise entre 0,7 et 1,5 µm. Après une désagglomération dans des conditions douces, la taille moyenne peut être d'au plus 1,5µm et avantageusement comprise entre 0,3 et 0,8µm.

L'invention comprend différents modes de réalisation.

Le premier mode correspond à la structure la plus simple de la composition. Dans ce cas, celle-ci ne comprend qu'un support avec un composé du zinc déposé sur le support. Selon une caractéristique de l'invention commune à tous les modes de réalisation, ce composé du zinc est obtenu par réaction d'un précurseur du zinc avec de l'ammoniaque et/ou un sel d'ammonium. Le composé du zinc formé par cette réaction peut ainsi être directement déposé sur le support. La forme sous laquelle se présente ce composé du zinc n'est pas connue précisément. Dans certains cas toutefois, on peut penser que le zinc est présent sous la forme d'un complexe zinc-ammoniaque de formule Zn(NH₃)ₓ(A)_{y} dans laquelle A représente un anion comme OH⁻, Cl⁻, l'anion acétate ou encore un mélange d'anions, x étant au plus égal à 4 et y égal à 2.

Dans le cas du deuxième mode de réalisation de l'invention, la composition comprend en outre une couche à base d'au moins un oxyde transparent, déposée à la surface du support. On pourra se référer aussi en ce qui conceme un produit de ce type comprenant une telle couche, à la demande de brevet français FR-A-2703999 au nom de la Demanderesse dont l'enseignement est incorporé ici.

Cette couche périphérique enrobant le support peut ne pas être parfaitement continue ou homogène. Toutefois, de préférence, les compositions selon ce mode de réalisation comprennent une couche de revêtement uniforme et d'épaisseur contrôlée d'oxyde transparent, et ceci de manière à ne pas altérer la couleur originelle du support avant enrobage.

Par oxyde transparent, on entend ici un oxyde qui, une fois déposé sur le support sous la forme d'une pellicule plus ou moins fine, n'absorbe que peu ou pas du tout les rayons lumineux dans le domaine du visible, et ceci de manière à ne pas ou peu masquer la couleur intrinsèque d'origine dudit support. En outre, il convient de noter que le terme oxyde, qui est utilisé par commodité dans l'ensemble de la présente description, doit être entendu comme couvrant également des oxydes du type hydraté.

Ces oxydes, ou oxydes hydratés, peuvent être amorphes et/ou cristallisés.

A titre d'exemple de tels oxydes, on peut plus particulièrement citer l'oxyde de silicium (silice), l'oxyde d'aluminium (alumine), l'oxyde de zirconium (zircone), l'oxyde de titane, le silicate de zirconium ZrSiO₄ (zircon) et les oxydes de terres rares. Selon une variante préférée, la couche enrobante est à base de silice. De manière encore plus avantageuse, cette couche est essentiellement, et de préférence uniquement, constituée de silice.

Selon un troisième mode de réalisation de l'invention, la composition peut contenir des atomes de fluor.

Dans ce cas, on pourra aussi se référer en ce qui conceme la disposition des atomes de fluor à la demande de brevet français FR-A-2706476 au nom de la Demanderesse dont l'enseignement est incorporé ici.

Les compositions fluorées peuvent présenter au moins l'une des caractéristiques suivantes :
- les atomes de fluor sont distribués selon un gradient de concentration décroissant de la surface au coeur desdites compositions.
- les atomes de fluor sont majoritairement répartis à la périphérie exteme des compositions. On entend ici par périphérie externe une épaisseur de matière mesurée à partir de la surface de la particule, de l'ordre de quelques centaines d'Angströms. On entend en outre par majoritairement que plus de 50% des atomes de fluor présents dans le sesquisulfure se trouvent dans ladite périphérie externe.
- le pourcentage en poids des atomes de fluor présents dans les compositions n'excède pas 10%, et de préférence 5%.
- les atomes de fluor sont présents sous la forme de composés fluorés ou sulfofluorés, en particulier sous la forme de fluorures de terres rares ou de sulfofluorures (thiofluorures) de terres rares.

Bien entendu, l'invention concerne aussi la combinaison des modes de réalisation qui ont été décrits précédemment. Ainsi, on peut envisager une composition comprenant un support avec une couche d'oxyde et comprenant en outre des atomes de fluor.

Dans tous les cas, le zinc est situé de préférence à la périphérie des particules qui constituent les compositions. En particulier, pour les compositions comprenant une couche d'oxyde sur le support, il peut être inclus dans la couche d'oxyde ou situé à la surface de celle-ci.

Le procédé de préparation des compositions de l'invention va maintenant être décrit.

Selon une première variante, le procédé est caractérisé en ce qu'on met en contact le support, un précurseur du zinc , de l'ammoniaque et/ou un sel d'ammonium et, le cas échéant, un précurseur de l'oxyde transparent et un agent fluorant, et on dépose le composé de zinc sur le support et, le cas échéant, on précipite l'oxyde transparent sur ledit support.

Selon une seconde variante, le traitement de fluoration se fait dans une première étape, puis, dans un deuxième temps, on met en contact le support ainsi traité, un précurseur du zinc , de l'ammoniaque et/ou un sel d'ammonium et, le cas échéant un précurseur de l'oxyde transparent, et on dépose le composé de zinc sur le support et, le cas échéant, on précipite l'oxyde transparent sur ledit support.

Une troisième variante du procédé est aussi envisageable. Dans ce cas, dans une première étape, on met en contact le support, un précurseur du zinc , de l'ammoniaque et/ou un sel d'ammonium et, le cas échéant un précurseur de l'oxyde transparent, et on dépose le composé de zinc sur le support et, le cas échéant, on précipite l'oxyde transparent sur ledit support puis, dans une deuxième étape, on effectue le traitement de fluoration.

Une autre variante du procédé est enfin possible. Dans ce cas, dans une première étape, on met en contact le support et un précurseur de l'oxyde transparent et on précipite l'oxyde transparent sur ledit support puis, dans une deuxième étape, on met en contact le support ainsi obtenu avec un précurseur du zinc, de l'ammoniaque et/ou un sel d'ammonium et on dépose le composé de zinc sur le support.

Dans le cas de cette dernière variante, on peut effectuer le traitement de fluoration lors de l'une des étapes précitées ou avant la première ou après la seconde.

En ce qui concerne d'une manière générale la formation de la couche d'oxyde sur le support et le traitement de fluoration, on pourra se reporter à l'enseignement des demandes de brevets mentionnées plus haut FR-A-2703999 et FR-A-2706476.

Plus précisément, dans le cas de la préparation d'une composition comprenant une couche d'oxyde transparent, le principe de préparation consiste donc essentiellement à précipiter l'oxyde sur le support. Des exemples de procédés vont être donnés ci-dessous pour les différents types d'oxydes, procédés dans lesquels le précurseur de l'oxyde peut être un alcoolate .

Dans le cas de la silice on peut mentionner la préparation de la silice par hydrolyse d'un alkyl-silicate, en formant un milieu réactionnel par mélange d'eau, d'alcool, du support qui est alors mis en suspension, et éventuellement d'une base, d'un fluorure alcalin ou d'un fluorure d'ammonium qui peut jouer le rôle de catalyseur de la condensation du silicate. On introduit ensuite l'alkyl-silicate. On peut encore effectuer une préparation par réaction du support, d'un silicate, du type silicate alcalin, et d'un acide.

Dans le cas d'une couche à base d'alumine, on peut faire réagir le support, un aluminate et un acide, ce par quoi on précipite de l'alumine. Cette précipitation peut aussi être obtenue en mettant en présence et en faisant réagir le support, un sel d'aluminium et une base.

Enfin, on peut former l'alumine par hydrolyse d'un alcoolate d'aluminium.

Pour ce qui est de l'oxyde de titane, on peut le précipiter en introduisant dans une suspension hydroalcoolique du support un sel de titane d'une part tel que TiCl₄, TiOCl₂ ou TiOSO₄, et une base d'autre part. On peut aussi opérer par exemple par hydrolyse d'un titanate d'alkyle ou précipitation d'un sol de titane.

Enfin, dans le cas d'une couche à base d'oxyde de zirconium, il est possible de procéder par cohydrolyse ou coprécipitation d'une suspension du support de cérium en présence d'un composé organométallique du zirconium, par exemple un alcoxyde de zirconium comme l'isopropoxyde de zirconium.

La fluoration peut être opérée selon toute technique connue en soi.

En particulier, l'agent de fluoration peut être liquide, solide ou gazeux. De préférence, on opère sous des conditions de traitement où l'agent de fluoration est liquide ou gazeux.

A titre d'exemples d'agents fluorants convenant pour la mise en oeuvre du traitement selon l'invention, on peut plus particulièrement citer le fluor F₂, les fluorures d'alcalins, le fluorure d'ammonium, les fluorures de gaz rares, le fluorure d'azote NF₃, le fluorure de bore BF₃, le tétrafluorométhane, l'acide fluorhydrique HF.

Dans le cas d'un traitement sous atmosphère fluorante, l'agent fluorant peut être utilisé pur ou en dilution dans un gaz neutre, par exemple de l'azote.

Les conditions de réaction sont choisies de préférence de manière telle que ledit traitement n'induise une fluoration qu'en surface du support (conditions douces). A cet égard, la conduite d'une fluoration jusqu'au coeur du support n'apporte pas de résultats substantiellement améliorés par rapport à une fluoration essentiellement de surface. D'une manière pratique, on peut suivre et contrôler expérimentalement le degré d'avancement de la réaction de fluoration, par exemple en mesurant l'évolution de la prise de masse des matériaux (prise de masse induite par l'introduction progressive du fluor).

Le précurseur du zinc peut être un oxyde ou un hydroxyde de zinc que l'on utilise en suspension. Ce précurseur peut être aussi un sel de zinc, de préférence un sel soluble. Ce peut être un sel d'acide inorganique comme un chlorure, ou encore un sel d'acide organique comme un acétate.

Selon une variante particulière de l'invention, l'agent de fluoration est le fluorure d'ammonium.

Il est aussi possible d'utiliser à la fois de l'ammoniaque et un sel d'ammonium.

Selon une caractéristique intéressante, la mise en contact entre le support, le précurseur du zinc , l'ammoniaque et/ou le sel d'ammonium et, le cas échéant, le précurseur de l'oxyde transparent et l'agent fluorant, se fait en présence d'un alcool. L'alcool utilisé est généralement choisi parmi les alcools aliphatiques tel que par exemple le butanol ou l'éthanol. L'alcool peut, en particulier, être apporté avec le précurseur du zinc sous forme d'une solution alcoolique de zinc.

Selon une autre variante intéressante de l'invention, on met en contact le support, le précurseur du zinc , l'ammoniaque et/ou le sel d'ammonium et, le cas échéant, le précurseur de l'oxyde transparent et l'agent fluorant, en présence d'un dispersant. Ce dispersant a pour but d'éviter l'agglomération des particules formant support lors de leur mise en suspension pour les traitements décrits ci-dessus. Il permet aussi de travailler dans des milieux plus concentrés. Il favorise la formation d'une couche homogène d'oxyde transparent sur l'ensemble des particules.

Ce dispersant peut être choisi dans le groupe des dispersants par effet stérique et notamment des polymères hydrosolubles ou organosolubles non ioniques. On peut citer comme dispersant la cellulose et ses dérivés, les polyacrylamides, les oxydes de polyéthylène, les polyéthylène glycols, les polyoxypropylène glycols polyoxyéthylénés, les polyacrylates, les alkyl phénols polyoxyéthylénés, les alcools à longues chaînes polyoxyéthylénés, les polyvinylalcools, les alkanolamides, les dispersants du type polyvinylpyrrolidone, les composés à base de gomme xanthane.

En outre, on peut noter qu'il peut être intéressant de traiter par ultrasons la suspension obtenue à partir du mélange des réactifs.

Enfin, le produit obtenu à la fin des opérations décrites ci-dessus, peut être lavé, à l'eau ou à l'alcool. Il peut être aussi séché à l'air ou encore sous vide.

La présente invention conceme aussi l'utilisation comme pigments colorants des compositions décrites plus haut ou obtenues par les procédés de préparation ci-dessus.

Les compositions ou produits de l'invention possèdent en effet un pouvoir de coloration et un pouvoir couvrant et, de ce fait, conviennent à la coloration de nombreux matériaux, tels que plastiques, peintures et autres. Ils sont tout particulièrement adaptés aux applications dans lesquelles ils sont mis en oeuvre à une température relativement élevée et dans des conditions où il y a un risque de dégagement d'H₂S par suite éventuellement d'une hydrolyse partielle du composé soufré.

Ainsi, et plus précisément, ils peuvent être utilisés dans la coloration de polymères pour matières plastiques qui peuvent être du type thermoplastiques ou thermodurcissables, ces polymères étant susceptibles de contenir des traces d'eau.

Comme résines thermoplastiques susceptibles d'être colorées selon l'invention, on peut citer, à titre purement illustratif, le chlorure de polyvinyle, l'alcool polyvinylique, le polystyrène, les copolymères styrène-butadiène, styrène-acrylonitrile, acrylonitrile-butadiène-styrène (A.B.S.), les polymères acryliques notamment le polyméthacrylate de méthyle, les polyoléfines telles que le polyéthylène, le polypropylène, le polybutène, le polyméthylpentène, les dérivés cellulosiques tels que par exemple l'acétate de cellulose, l'acéto-butyrate de cellulose, l'éthylcéllulose, les polyamides dont le polyamide 6-6.

Concemant les résines thermodurcissables pour lesquelles les compositions selon l'invention conviennent également, on peut citer, par exemple, les phénoplastes, les aminoplastes notamment les copolymères urée-formol, mélamine-formol, les résines époxy et les polyesters thermodurcissables.

On peut également mettre en oeuvre les compositions de l'invention dans des polymères spéciaux tels que des polymères fluorés en particulier le polytétrafluoréthylène (P.T.F.E.), les polycarbonates, les élastomères silicones, les polyimides.

Dans cette application spécifique pour la coloration des plastiques, on peut mettre en oeuvre les compositions de l'invention directement sous forme de poudres. On peut également, de préférence, les mettre en oeuvre sous une forme pré-dispersée, par exemple en prémélange avec une partie de la résine, sous forme d'un concentré pâte ou d'un liquide ce qui permet de les introduire à n'importe quel stade de la fabrication de la résine.

Ainsi, les produits selon l'invention peuvent être incorporés dans des matières plastiques telles que celles mentionnées ci-avant dans une proportion pondérale allant généralement soit de 0,01 à 5% (ramenée au produit final) soit de 20 à 70% dans le cas d'un concentré.

Les produits de l'invention peuvent être également utilisés dans le domaine des peintures et lasures et plus particulièrement dans les résines suivantes : résines alkydes dont la plus courante est dénommée glycérophtalique; les résines modifiées à l'huile longue ou courte; les résines acryliques dérivées des esters de l'acide acrylique (méthylique ou éthylique) et méthacrylique éventuellement copolymérisés avec l'acrylate d'éthyle, d'éthyl-2 hexyle ou de butyle; les résines vinyliques comme par exemple l'acétate de polyvinyle, le chlorure de polyvinyle, le butyralpolyvinylique, le formalpolyvinylique, et les copolymères chlorure de vinyle et acétate de vinyle ou chlorure de vinylidène; les résines aminoplastes ou phénoliques le plus souvent modifiées; les résines polyesters; les résines polyuréthannes; les résines époxy; les résines silicones.

Généralement, les produits sont mis en oeuvre à raison de 5 à 30% en poids de la peinture, et de 0,1 à 5% en poids du lasure.

Enfin, les produits selon l'invention sont également susceptibles de convenir pour des applications dans l'industrie du caoutchouc, notamment dans les revêtements pour sols, dans l'industrie du papier et des encres d'imprimerie, dans le domaine de la cosmétique, ainsi que nombreuses autres utilisations comme par exemple, et non limitativement, les teintures, dans les cuirs pour le finissage de ceux-ci et les revêtements stratifiés pour cuisines et autres plans de travail, les céramiques et les glaçures.

Les produits de l'invention peuvent aussi être utilisés dans la coloration des matériaux à base de ou obtenu à partir d'au moins un liant minéral.

Ce liant minéral peut être choisi parmi les liants hydrauliques, les liants aériens, le plâtre et les liants du type sulfate de calcium anhydre ou partiellement hydraté.

Par liants hydrauliques, on entend les substances ayant la propriété de faire prise et de durcir après addition d'eau en formant des hydrates insolubles dans l'eau. Les produits de l'invention s'appliquent tout particulièrement à la coloration des ciments et bien entendu des bétons fabriqués à partir de ces ciments par addition à ceux-ci d'eau, de sable et/ou de graviers.

Dans le cadre de la présente invention, le ciment peut, par exemple, être du type alumineux. On entend par là tout ciment contenant une proportion élevée soit d'alumine en tant que telle soit d'aluminate soit des deux. On peut citer à titre d'exemple les ciments à base d'aluminate de calcium, notamment ceux du type SECAR.

Le ciment peut aussi être du type silicate et plus particulièrement à base de silicate de calcium. On peut donner à titre d'exemple les ciments PORTLAND et, dans ce type de ciments, les Portland à prise rapide ou très rapide, les ciments blancs, ceux résistant aux sulfates ainsi que ceux comprenant des laitiers de hauts-fourneaux et/ou des cendres volantes et/ou du méta-kaolin.

On peut aussi mentionner les ciments à base d'hémihydrate, de sulfate de calcium ainsi que les ciments magnésiens dits ciments de Sorel.

Les produits de l'invention s'utilisent aussi à la coloration des liants aériens, c'est à dire des liants durcissant à l'air libre par l'action du CO₂, du type oxyde ou hydroxyde de calcium ou de magnésium.

Les produits de l'invention s'utilisent enfin à la coloration du plâtre et des liants du type sulfate de calcium anhydre ou partiellement hydraté (CaSO₄ et CaSO₄, 1/2H₂O).

Enfin, l'invention concerne des compositions de matière colorées notamment du type plastiques, peintures, lasures, caoutchoucs, céramiques, glaçures, papiers, encres, produits cosmétiques, teintures, cuirs, revêtements stratifiés ou du type à base ou obtenu à partir d'au moins un liant minéral, qui comprennent comme pigment colorant, une composition selon l'invention ou obtenue par un procédé du type décrit ci-dessus.

Des exemples vont maintenant être donnés.

### EXEMPLES

### Tests de mesure de relargage d'H₂S par le pigment :

Deux tests sont donnés ci-dessous.

### Test 1

On pèse 15g de polyamide (PA) 6/6 chargé à plus de 1% d'humidité et 0,375g de pigment (2,5%) que l'on introduit dans une cellule électrochimique à double enveloppe munie d'un couvercle et thermostatée à 75°C.

On introduit alors à l'aide d'une pipette volumétrique 0,13ml d'eau permutée dans le réacteur dont on étanchéifie les entrées à l'aide de parafilm®.

On malaxe le mélange réactionnel à l'aide d'une pale Rushton tournant à 700tr/min pendant 10min.

On prélève à l'aide d'une pompe d'échantillonnage (GASTEC NT 00053A) et d'un tube indicateur colorimétrique (GASTEC N°4LL, PROLABO) 100ml de l'atmosphère réactionnelle en perçant le parafilm® à l'aide du tube.

On laisse réagir le tube pendant une minute conformément à son mode d'utilisation puis on lit directement sur le tube la teneur en H₂S dans l'enceinte réactionnelle (en ppm). La précision est de 5ppm.

### Test 2

On introduit dans la cellule électrochimique précédente 0,45ml d'eau bipermutée à l'aide d'une pipette volumétrique. On ajoute ensuite 10g de polypropylène en poudre contenant 0,5g de pigment (5%) dans le réacteur dont on étanchéifie les entrées à l'aide de parafilm.

On procède ensuite comme pour le test 1.

Le test 2 est considéré comme plus dur que le test 1. Ceci est du au fait de la présence d'une plus grande quantité d'eau et de pigment. Cette plus grande quantité de pigment augmente les risques d'abrasion du pigment par lui-même. L'utilisation de polypropylène durcit aussi le test car ce polymère est plus abrasif que le polyamide.

### Coordonnées chromatiques

Les coordonnées chromatiques L*, a* et b* sont données dans le système CIE 1976 (L*, a* et b*) tel que défini par la Commission Internationale d'Eclairage et répertorié dans le Recueil des Normes Françaises (AFNOR), couleur colorimétrique n° X08-12, X08-14 (1983). Elles sont déterminées pour ce qui concerne les mesures faites sur les produits et les plastiques au moyen d'un colorimètre commercialisé par la Société Pacific Scientific. La nature de l'illuminant est le D65. La surface d'observation est une pastille circulaire de 12,5 cm² de surface. Les conditions d'observations correspondent à une vision sous un angle d'ouverture de 10°. Dans les mesures données, la composante spéculaire est exclue.

### Injection dans le plastique :

Le produit est incorporé dans une proportion en poids de 0,5% dans du polypropylène. On forme des éprouvettes par injection à 240°C.

La mesure des coordonnées chromatiques est effectuée sur fond blanc.

### Conditions opératoires :

La polyvinylpyrrolidone (PVP) est dissoute dans l'éthanol.

A cette solution est ajouté le sulfure de cérium fluoré ou non. La suspension ainsi obtenue est dispersée à l'aide d'ultrasons et on ajoute ensuite la solution d'ammoniaque, puis le précurseur du zinc dissous dans l'éthanol. Le silicate d'éthyle est introduit de façon continue pendant deux heures. Après la fin de l'introduction du silicate d'éthyle, on effectue un temps de mûrissement de deux heures. Les particules ainsi obtenues sont lavées à l'éthanol par filtration puis séchées à 50°C pendant douze heures sauf indications contraires.

### EXEMPLE 1 :

On utilise les réactifs dans les proportions suivantes :

| | g/kg |
|---|---|
| Sulfure de cérium | 200 |
| Ethanol 95% | 643 |
| Ammoniaque (32%) | 100 |
| Acétate de zinc | 20 |
| Silicate d'éthyle | 32 |
| PVP K10 (Société Aldrich) | 5 |
| M=10000 | |

Le sulfure de cérium utilisé est un sulfure de structure γ cubique, dopé au lithium dans un rapport atomique Li/Ce de 0,1. Ce sulfure a été préalablement fluoré de la manière suivante. 10 g de produit sont introduits dans 100 ml d'une solution de fluorure d'ammonium (5% en masse).

Par ajout d'une solution d'ammoniaque, on porte le pH du mélange à 8 et on laisse le milieu sous agitation pendant une heure. Le produit est ensuite filtré, puis séché dans un dessiccateur sous vide.

On traite le produit ainsi obtenu en mettant en oeuvre les conditions opératoires données plus haut en utilisant l'ammoniaque.

Le produit obtenu présente les coordonnées chromatiques suivantes :
L*= 45,4; a*= 54,2; b*= 43,6

On obtient les valeurs suivantes après injection dans le polypropylène :
L* = 42,3; a* = 49,4; b* = 34,9

La mesure du relargage d'H₂S donne les résultats suivants :
Test 1 : non détecté; test 2 : non détecté
H₂S libéré après injection dans le polypropylène à 240°C : non détecté.

### EXEMPLE 2 COMPARATIF

On reproduit l'exemple 1 mais sans addition d'acétate de zinc

Le produit obtenu présente les coordonnées chromatiques suivantes :
L*= 45,7; a*= 49,8; b*= 35,6

On obtient les valeurs suivantes après injection dans le polypropylène :
L* = 43,5; a* = 49,8; b* = 36,9

La mesure du relargage d'H₂S donne les résultats suivants :
Test 1 : 20ppm; test 2 : >60ppm (supérieur à la limite de détection)
H₂S libéré après injection dans le polypropylène à 240°C : 10ppm.

### EXEMPLE 3

On utilise les réactifs dans les proportions suivantes :

| | g/kg |
|---|---|
| Sulfure de cérium | 200 |
| Ethanol 95% | 647,6 |
| Ammoniaque (32%) | 100 |
| Chlorure de zinc | 15,4 |
| Silicate d'éthyle | 32 |
| PVP K40 ( Société Aldrich) | 5 |
| M=40000 | |

Le sulfure de cérium est le sulfure fluoré utilisé dans l'exemple 1.

Le produit obtenu présente les coordonnées chromatiques suivantes :
L*= 47,7; a*= 52,4; b*= 39

On obtient les valeurs suivantes après injection dans le polypropylène :
L* = 43,8; a* = 51,3; b* = 39,8

La mesure du relargage d'H₂S donne les résultats suivants :
Test 1 : non détecté. Test 2: non détecté.
H₂S libéré après injection dans le polypropylène à 240°C : non détecté.

### EXEMPLE 4

On procède comme dans l'exemple 3 mais le produit est séché sous vide à 180°C pendant 5 heures.

On obtient les résultats suivants :
Test 1 : non détecté. Test 2: non détecté.
H₂S libéré après injection dans le polypropylène à 240°C : non détecté.
Les coordonnées chromatiques restent inchangées.

### EXEMPLE 5

On procède comme dans l'exemple 1 en remplaçant le sulfure de cérium par la même quantité de pigment Ultramarine Bleu.

La mesure du relargage d'H₂S donne les résultats suivants :
Test 1 : non détecté. Test 2: non détecté.

### EXEMPLE 6 COMPARATIF

On procède comme dans l'exemple 5 mais sans addition d'acétate de zinc.

La mesure du relargage d'H₂S donne les résultats suivants :
Test 1 : 20ppm. Test 2 : 30ppm.

### EXEMPLE 7 COMPARATIF

Cet exemple concerne la préparation d'un produit pour lequel le zinc n'est pas déposé sur le support par réaction avec de l'ammoniaque.

On part du sulfure de structure γ cubique, dopé au lithium et fluoré, décrit dans l'exemple 1. On traite ce produit en mettant en oeuvre les conditions opératoires données plus haut en introduction des exemples mais sans sel de zinc et en utilisant de l'ammoniaque dans les proportions suivantes :

| | g/kg |
|---|---|
| Sulfure de cérium | 200 |
| Ethanol 95% | 653 |
| Ammoniaque (32%) | 100 |
| Silicate d'éthyle | 32 |
| PVP K10 | 5 |

On obtient ainsi un pigment présentant une couche de silice et des atomes de fluor.

Ce pigment est introduit ensuite dans une solution éthanolique à raison de 100g/l contenant du stéarate de zinc. La proportion stéarate/pigment est de 10% en poids.

Le produit est ensuite séparé et séché à 50°C pendant 12 heures.

La mesure du relargage d'H₂S selon le test 2 donne une valeur de 30ppm.

### EXEMPLE 8

On procède comme dans l'exemple 1, mais le précurseur de zinc est l'oxyde de zinc, dispersé préalablement dans de l'éthanol, avant ajout.

On utilise les réactifs dans les proportions suivants :

| | g/kg |
|---|---|
| Sulfure de cérium | 200 |
| EtOH 95% | 643 |
| Ammoniaque 32% | 100 |
| Oxyde de zinc | 20 |
| Silicate d'éthyle | 32 |
| PVP K 10 | 5 |

Le produit obtenu présente les coordonnées chromatiques suivantes :
- L = 45, 7
- a* = 48,9
- b* = 34,2

On obtient les valeurs suivantes après injection dans le polypropylène :
- L = 39
- a* = 49
- b* = 37

La mesure de relargage d'H₂S donne les résultats suivants :
- Test 1 = Test 2 = non détecté.

### EXEMPLE 9

On procède comme dans l'exemple 1, mais le précurseur de zinc est l'oxyde de zinc, dispersé préalablement dans de l'éthanol, avant ajout.

On utilise les réactifs dans les proportions suivants :

| | g/kg |
|---|---|
| CeLaS₃ | 200 |
| EtOH 95% | 643 |
| Ammoniaque 32% | 100 |
| Oxyde de zinc | 20 |
| Silicate d'éthyle | 32 |
| PVP K 10 | 5 |

Le produit obtenu présente les coordonnées chromatiques suivantes :
- L = 65,9
- a* = 49,1
- b* = 64

On obtient les valeurs suivantes après injection dans le polypropylène :
- L = 61,5
- a* = 46,5
- b* = 67,3

La mesure de relargage d'H₂S donne les résultats suivants :
Test 1 = Test 2 = non détecté.

## Revendications

1. Composition caractérisée en ce qu'elle comprend :
- un support à base d'au moins un composé soufré;
- un composé du zinc, déposé sur le support et obtenu par réaction d'un précurseur du zinc avec de l'ammoniaque et/ou un sel d'ammonium.

2. Composition selon la revendication 1, caractérisée en ce qu'elle comprend en outre une couche à base d'au moins un oxyde transparent déposée à la surface du support.

3. Composition selon la revendication 1 ou 2, caractérisée en ce qu'elle comprend en outre des atomes de fluor.

4. Composition selon l'une des revendications précédentes, caractérisée en ce que le composé soufré est un sulfate ou un sulfure, plus particulièrement un sulfure de terre rare.

5. Composition selon la revendication 4, caractérisée en ce que le sulfure est un sulfure de terre rare et d'alcalin.

6. Composition selon la revendication 4, caractérisée en ce que le sulfure est un sesquisulfure de terre rare qui contient au moins un élément alcalin et/ou alcalino-terreux dont une partie au moins est incluse dans le réseau cristallin dudit sesquisulfure.

7. Composition selon la revendication 6, caractérisée en ce que le sesquisulfure de terre rare est un sesquisulfure Ce₂S₃ γ cubique.

8. Composition selon l'une des revendications 2 à 7, caractérisée en ce que l'oxyde transparent est la silice.

9. Composition selon l'une des revendications 3 à 8, caractérisée en ce que les atomes de fluor sont distribués selon un gradient décroissant de la surface au coeur de la composition.

10. Procédé de préparation d'une composition selon l'une des revendications précédentes, caractérisé en ce qu'on met en contact le support, un précurseur du zinc, de l'ammoniaque et/ou un sel d'ammonium et, le cas échéant, un précurseur de l'oxyde transparent et un agent fluorant, et on dépose le composé de zinc sur le support et, le cas échéant, on précipite l'oxyde transparent sur ledit support.

11. Procédé de préparation d'une composition selon l'une des revendications 2 à 9, caractérisé en ce qu'on soumet le support à un traitement de fluoration dans un premier temps, puis, dans un deuxième temps, on met en contact ledit support ainsi traité, un précurseur du zinc, de l'ammoniaque et/ou un sel d'ammonium et, le cas échéant un précurseur de l'oxyde transparent, et on dépose le composé de zinc sur le support et, le cas échéant, on précipite l'oxyde transparent sur ledit support.

12. Procédé de préparation d'une composition selon l'une des revendications 2 à 9, caractérisé en ce que, dans une première étape, on met en contact le support, un précurseur du zinc, de l'ammoniaque et/ou un sel d'ammonium et, le cas échéant un précurseur de l'oxyde transparent, et on dépose le composé de zinc sur le support et, le cas échéant, on précipite l'oxyde transparent sur ledit support puis, dans une deuxième étape, on effectue le traitement de fluoration.

13. Procédé de préparation d'une composition selon l'une des revendications 2 à 9, caractérisé en ce que, dans une première étape, on met en contact le support et un précurseur de l'oxyde transparent et on précipite l'oxyde transparent sur ledit support puis, dans une deuxième étape, on met en contact le support ainsi obtenu avec un précurseur du zinc, de l'ammoniaque et/ou un sel d'ammonium et on dépose le composé de zinc sur le support.

14. Procédé selon la revendication 13, caractérisé en ce qu'on effectue le traitement de fluoration lors de l'une des étapes précitées ou avant la première ou après la seconde.

15. Procédé selon l'une des revendications 10 à 14, caractérisé en ce que le précurseur de l'oxyde est un alcoolate.

16. Procédé selon l'une des revendications 10 à 15, caractérisé en ce qu'on utilise comme agent fluorant ou pour le traitement de fluoration précité, le fluorure d'ammonium.

17. Procédé selon l'une des revendications 10 à 16, caractérisé en ce qu'on met en contact le support, le précurseur du zinc, l'ammoniaque et/ou le sel d'ammonium et, le cas échéant, le précurseur de l'oxyde transparent et l'agent fluorant, en présence d'un alcool.

18. Procédé selon l'une des revendications 10 à 16, caractérisé en ce qu'on met en contact le support, le précurseur du zinc, l'ammoniaque et/ou le sel d'ammonium et, le cas échéant, le précurseur de l'oxyde transparent et l'agent fluorant, en présence d'un dispersant.

19. Procédé selon la revendication 18, caractérisé en ce que le dispersant est choisi parmi les dispersants par effet stérique et notamment les polymères hydrosolubles ou organosolubles non ioniques et plus particulièrement ceux du type polyvinylpyrrolidone.

20. Utilisation d'une composition selon l'une des revendications 1 à 9 ou obtenue par un procédé selon l'une des revendications 10 à 19, comme pigment colorant.

21. Utilisation selon la revendication 20, caractérisée en ce que la composition est employée comme pigment dans des matières plastiques, des peintures, des lasures, des caoutchoucs, des céramiques, des glaçures, des papiers, des encres, des produits cosmétiques, des teintures, des cuirs, des revêtements stratifiés et des matériaux à base ou obtenus à partir d'au moins un liant minéral.

22. Compositions de matière colorées notamment du type plastiques, peintures, lasures, caoutchoucs, céramiques, glaçures, papiers, encres, produits cosmétiques, teintures, cuirs, revêtements stratifiés ou du type à base ou obtenus à partir d'au moins un liant minéral, caractérisées en ce qu'elles comprennent, comme pigment colorant, une composition selon l'une des revendications 1 à 9 ou obtenue par un procédé selon l'une des revendications 10 à 19.

## Patentansprüche

1. Zusammensetzung enthaltend:
- einen Träger auf Basis mindestens einer Schwefelverbindung;
- eine auf dem Träger aufgebrachte Zinkverbindung, die man durch die Reaktion eines Ausgangsstoffes für das Zink mit Ammoniak und/oder einem Ammoniaksalz erhält.

2. Zusammensetzung nach Anspruch 1, dadurch gekennzeichnet, daß sie außerdem eine Schicht auf Basis mindestens eines auf der Trägeroberfläche aufgebrachten durchsichtigen Oxids enthält.

3. Zusammensetzung nach Anspruch 1 oder 2, dadurch gekennzeichnet, daß sie außerdem Fluoratome enthält.

4. Zusammensetzung nach einem der vorangehenden Ansprüche, dadurch gekennzeichnet, daß die Schwefelverbindung ein Sulfat oder Sulfid, insbesondere ein Sulfid eines Metalls der Seltenen Erde ist.

5. Zusammensetzung nach Anspruch 4, dadurch gekennzeichnet, daß das Sulfid ein Lanthanoid- und Alkalisulfid ist.

6. Zusammensetzung nach Anspruch 4, dadurch gekennzeichnet, daß das Sulfid ein Lanthanoidsesquisulfid ist, das mindestens ein Alkali- und/oder Erdalkalielement aufweist, von denen mindestens ein Teil im Kristallgitter des genannten Sesquisulfids eingeschlossen ist.

7. Zusammensetzung nach Anspruch 6, dadurch gekennzeichnet, daß das Lanthanoidsesquisulfid ein γ-kubisches Ce₂S₃-Sesquisulfid ist.

8. Zusammensetzung nach einem der Ansprüche 2 bis 7, dadurch gekennzeichnet, daß das durchsichtige Oxid Kieselsäure ist.

9. Zusammensetzung nach einem der Ansprüche 3 bis 8, dadurch gekennzeichnet, daß die Fluoratome gemäß einem von der Oberfläche zum Inneren der Zusammensetzung abnehmenden Gradienten verteilt sind.

10. Verfahren zur Herstellung einer Zusammensetzung nach einem der vorangehenden Ansprüche, dadurch gekennzeichnet, daß man einen Träger mit einem Zinkausgangsstoff, Ammoniak und/oder einem Ammoniaksalz, gegebenenfalls einem Ausgangsstoff für ein durchsichtiges Oxid und einem Fluorierungsmittel zusammenbringt, die Zinkverbindung auf den Träger aufbringt und gegebenenfalls das durchsichtige Oxid auf dem genannten Träger niederschlägt.

11. Verfahren zur Herstellung einer Zusammensetzung nach einem der Ansprüche 2 bis 9, dadurch gekennzeichnet, daß man den Träger in einem ersten Schritt einer Fluorierung unterzieht, danach in einem zweiten Schritt den genannten so behandelten Träger mit einem Zinkausgangsstoff, Ammoniak und/oder einem Ammoniaksalz und gegebenenfalls einem Ausgangsstoff für ein durchsichtiges Oxid zusammenbringt, die Zinkverbindung auf den Träger aufbringt und gegebenenfalls das durchsichtige Oxid auf dem genannten Träger niederschlägt.

12. Verfahren zur Herstellung einer Zusammensetzung nach einem der Ansprüche 2 bis 9, dadurch gekennzeichnet, daß man in einem ersten Schritt den Träger mit einem Zinkausgangsstoff, Ammoniak und/oder einem Ammoniaksalz und gegebenenfalls einem Ausgangsstoff für ein durchsichtiges Oxid zusammenbringt, die Zinkverbindung auf den Träger aufbringt, gegebenenfalls das durchsichtige Oxid auf dem genannten Träger niederschlägt und dann in einem zweiten Schritt die Fluorierung durchführt.

13. Verfahren zur Herstellung einer Zusammensetzung nach einem der Ansprüche 2 bis 9, dadurch gekennzeichnet, daß man in einem ersten Schritt den Träger und einen Ausgangsstoff für ein durchsichtiges Oxid zusammenbringt, das durchsichtige Oxid auf dem genannten Träger niederschlägt, dann in einem zweiten Schritt den so erhaltenen Träger mit einem Zinkausgangsstoff, Ammoniak und/oder einem Ammoniaksalz zusammenbringt und die Zinkverbindung auf den Träger aufbringt.

14. Verfahren nach Anspruch 13, dadurch gekennzeichnet, daß man die Fluorierung während eines der genannten Schritte oder vor dem ersten oder nach dem zweiten durchführt.

15. Verfahren nach einem der Ansprüche 10 bis 14, dadurch gekennzeichnet, daß der Oxidausgangsstoff ein Alkoholat ist.

16. Verfahren nach einem der Ansprüche 10 bis 15, dadurch gekennzeichnet, daß man als Fluorierungsmittel oder zur genannten Fluorierung Ammoniumfluorid verwendet.

17. Verfahren nach einem der Ansprüche 10 bis 16, dadurch gekennzeichnet, daß man den Träger mit dem Zinkausgangsstoff, Ammoniak und/oder dem Ammoniaksalz, gegebenenfalls dem Ausgangsstoff für ein durchsichtiges Oxid und dem Fluorierungsmittel in Gegenwart eines Alkohols zusammenbringt.

18. Verfahren nach einem der Ansprüche 10 bis 16, dadurch gekennzeichnet, daß man den Träger mit dem Zinkausgangsstoff, Ammoniak und/oder dem Ammoniaksalz, gegebenenfalls dem Ausgangsstoff für ein durchsichtiges Oxid und dem Fluorierungsmittel in Gegenwart eines Dispersionsmittels zusammenbringt.

19. Verfahren nach Anspruch 18, dadurch gekennzeichnet, daß das Dispersionsmittel aus den sterischen Dispersionsmitteln, insbesondere den wasser- oder organolöslichen nichtionischen Polymeren und noch spezieller den Polyvinylpyrrolidonen, gewählt ist.

20. Verwendung einer Zusammensetzung nach einem der Ansprüche 1 bis 9 oder erhalten durch ein Verfahren nach einem der Ansprüche 10 bis 19 als Farbpigment.

21. Verwendung nach Anspruch 20, dadurch gekennzeichnet, daß die Zusammensetzung als Pigment in Kunststoffen, Farben, Lasuren, Kautschuk, Keramik, Glasuren, Papier, Tinte, kosmetischen Produkten, Beizen, Leder, mehrlagigen Überzügen sowie in Materialien auf Basis oder erhalten aus mindestens einem anorganischen Binder verwendet wird.

22. Zusammensetzungen aus gefärbten Stoffen, insbesondere Plastik, Farben, Lasuren, Kautschuk, Keramik, Glasuren, Papier, Tinte, kosmetischen Produkten, Beizen, Leder, mehrlagigen Überzügen oder Materialien auf Basis oder erhalten aus mindestens einem anorganischen Binder, dadurch gekennzeichnet, daß sie als Farbpigment eine Zusammensetzung nach einem der Ansprüche 1 bis 9 oder erhalten durch ein Verfahren nach einem der Ansprüche 10 bis 19 enthalten.

## Claims

1. A composition, characterized in that it comprises:
• a support based on at least one sulphur-containing compound;
• a zinc compound, deposited on the support and obtained by reacting a zinc precursor with ammonia and/or an ammonium salt.

2. A composition according to claim 1, characterized in that it further comprises a layer based on at least one transparent oxide deposited on the support surface.

3. A composition according to claim 1 or claim 2, characterized in that it further comprises fluorine atoms.

4. A composition according to any one of the preceding claims, characterized in that the sulphur-containing compound is a sulphate or a sulphide, more particularly a rare earth sulphide.

5. A composition according to claim 4, characterized in that the sulphide is a sulphide of a rare earth and an alkali.

6. A composition according to claim 4, characterized in that the sulphide is a rare earth sesquisulphide which contains at least one alkali and/or alkaline-earth element at least a portion of which is included in the crystalline framework of said sesquisulphide.

7. A composition according to claim 6, characterized in that the rare earth sesquisulphide is a γ cubic Ce₂S₃ sesquisulphide.

8. A composition according to any one of claims 2 to 7, characterized in that the transparent oxide is silica.

9. A composition according to any one of claims 3 to 8, characterized in that the fluorine atoms are distributed in accordance with a gradient which decreases from the surface to the core of the composition.

10. A process for preparing a composition according to any one of the preceding claims, characterized in that the support, a zinc precursor, ammonia and/or an ammonium salt and, if necessary, a transparent oxide precursor and a fluorinating agent are brought into contact and the zinc compound is deposited on the support and, if necessary, the transparent oxide is precipitated onto said support.

11. A process for preparing a composition according to any one of claims 2 to 9, characterized in that the support first undergoes a fluorination treatment then secondly said treated support is brought into contact with a zinc precursor, ammonia and/or an ammonium salt and, if necessary, a transparent oxide precursor, the zinc compound is deposited on the support and, if necessary, the transparent oxide is precipitated onto said support.

12. A process for preparing a composition according to any one of claims 2 to 9, characterized in that in a first step the support is brought into contact with a zinc precursor, ammonia and/or an ammonium salt and, if necessary, a transparent oxide precursor, the zinc compound is deposited on the support and, if necessary, the transparent oxide is precipitated onto said support, then in a second step, the fluorination treatment is carried out.

13. A process for preparing a composition according to any one of claims 2 to 9, characterized in that in a first step the support is brought into contact with a transparent oxide precursor and the transparent oxide is precipitated onto said support, then in a second step, the support obtained is brought into contact with a zinc precursor, ammonia and/or an ammonium salt and the zinc compound is deposited onto the support.

14. A process according to claim 13, characterized in that the fluorination treatment is carried out during one of the steps defined above or before the first or after the second step.

15. A process according to any one of claims 10 to 14, characterized in that the oxide precursor is an alcoholate.

16. A process according to any one of claims 10 to 15, characterized in that the agent used as the fluorinating agent or for the fluorination treatment defined above is ammonium fluoride.

17. A process according to any one of claims 10 to 16, characterized in that the support, the zinc precursor, ammonia and/or ammonium salt and, if necessary the transparent oxide precursor and the fluorinating agent, are brought into contact in the presence of an alcohol.

18. A process according to any one of claims 10 to 16, characterized in that the support, zinc precursor, ammonia and/or ammonium salt and, if necessary, the transparent oxide precursor and the fluorinating agent are brought into contact in the presence of a dispersing agent.

19. A process according to claim 18, characterized in that the dispersing agent is selected from steric effect dispersing agents, in particular non ionic organosoluble or hydrosoluble polymers, more particularly of the polyvinylpyrrolidone type.

20. Use of a composition according to any one of claims 1 to 9 or obtained by a process according to any one of claims 10 to 19, as a coloration pigment.

21. Use according to claim 20, characterized in that the composition is used as a pigment in plastics materials, paints, finishes, rubbers, ceramics, glazes, papers, inks, cosmetic products, dyes, leathers, laminated coatings and materials based on or obtained from at least one mineral binder.

22. Coloured compositions, in particular plastics materials, paints, finishes, rubbers, ceramics, glazes, papers, inks, cosmetic products, dyes, leathers or laminated coatings or materials based on or obtained from at least one mineral binder, characterized in that they comprise, as a colouring pigment, a composition according to any one of claims 1 to 9 or obtained by a process according to any one of claims 10 to 19.
